# EUROPEAN PATENT SPECIFICATION

(11) **EP 2 114 921 B1**
(45) Date of publication and mention of the grant of the patent: **26.12.2012**
(21) Application number: 07858212.9
(22) Date of filing: 28.12.2007
(51) Int. Cl.: C07D 401/14, A61K 31/444, A61P 25/00, A61P 7/00, A61P 9/00

(54) **SUBSTITUTED OXINDOLE DERIVATIVE AND ITS USE AS A VASOPRESSIN RECEPTOR LIGAND**
SUBSTITUIERTES OXINDOLDERIVAT UND DESSEN VERWENDUNG ALS VASOPRESSINREZEPTORLIGAND
DÉRIVÉ D'OXINDOLE SUBSTITUÉ ET SON UTILISATION COMME LIGAND DU RÉCEPTEUR DE LA VASOPRESSINE

(30) Priority: 30.12.2006 DE 102006062508
(43) Date of publication of application: 11.11.2009
(73) Proprietor: Abbott GmbH & Co. KG, 65205 Wiesbaden (DE)
(72) Inventor: NETZ, Astrid, 67061 Ludwigshafen (DE); OOST, Thorsten, 88400 Biberach An Der Riss (DE); GENESTE, Hervé, 67061 Ludwigshafen (DE); BRAJE, Wilfried Martin, 67061 Ludwigshafen (DE); WERNET, Wolfgang, 67061 Ludwigshafen (DE); UNGER, Liliane, 67061 Ludwigshafen (DE); HORNBERGER, Wilfried, 67061 Ludwigshafen (DE); LUBISCH, Wilfried, 69118 Heidelberg (DE)
(74) Representative: Reitstötter - Kinzebach
(86) International application number: PCT/EP2007/064619
(87) International publication number: WO 2008/080970

(56) References cited:
- WO-A-2005/030755
- WO-A-2006/005609
- WO-A-2006/100081
- WO-A-2006/100082

## Description

The present invention relates to a novel substituted oxindole derivative, to medicaments comprising it and to its use for treating diseases.

Vasopressin is an endogenous hormone which exerts widely diverse effects on organs and tissues. It is suspected that the vasopressin system is involved in various pathological states such as, for example, heart failure and high blood pressure. At present three receptors (V1a, V1b or V3 and V2) via which vasopressin mediates its numerous effects are known. Antagonists of these receptors are therefore being investigated as possible novel therapeutic approaches to the treatment of diseases (M. Thibonnier, Exp.Opin. Invest. Drugs 1998, 7(5), 729-740).

The present application describes novel substituted oxindoles carrying an arylsulphonyl group in position 1. 1-Phenylsulphonyl-1,3-dihydro-2*H*-indol-2-ones have previously been described as ligands of vasopressin receptors. WO 93/15051, WO95/18105, WO 98/25901, WO 01/55130, WO 01/55134, WO 01/64668 and WO 01/98295 describe derivatives derived from the oxindole skeleton and having arylsulphonyl groups in position 1. These compounds differ essentially in the substitution in position 3.

In particular, WO 93/15051 and WO 98/25901 describe 1-phenylsulphonyl-1,3-dihydro-2H-indol-2-ones in which the oxindole structure is substituted in position 3 by two alkyl radicals which may also together form a cycloalkyl radical (spiro linkage) as ligands of vasopressin receptors. Alternatively, the spiro ring may comprise heteroatoms, such as oxygen and nitrogen (optionally with substituents).

WO 95/18105 describes 1-phenylsulphonyl-1,3-dihydro-2H-indol-2-ones having a nitrogen atom in position 3 as ligands of vasopressin receptors. In addition, radicals which are selected from the group consisting of alkyl, cycloalkyl, phenyl and benzyl are attached in position 3 (in each case optionally with substituents).

WO 03/008407 describes 1-phenylsulphonyloxindoles in which pyridylpiperazines are attached via an oxycarbonyl group to the oxindole in position 3.

WO 2005/030755 describes as Example 108, the carbamate compound 4-(1-methylpiperidin-4-yl)piperazine-1-carboxylic acid 5-cyano-1-(2,4-dimethoxy-phenylsulphonyl)-3-(2-methoxypyridin-3-yl)-2-oxo-2,3-Dihydro-1H-indol-3-yl ester (according to IUPAC nomenclature: 5-cyano-1-[(2,4-dimethoxyphenyl)sulphonyl]-3-(2-methoxypyridin-3-yl)-2-oxo-2,3-dihydro-1*H*-indol-3-yl 4-(1-methylpiperidin-4-yl)piperazine-1-carboxylate).

WO 06/005609 describes the 2-ethoxyphenyl urea compounds *N*-[5-cyano-1-[(2,4-dimethoxyphenyl)sulphonyl]-3-(2-ethoxyphenyl)-2-oxo-2,3-dihydro-1*H*-indol-3-yl]-4-(1-methylpiperidin-4-yl)piperazine-1-carboxamide (as Example 119) and *N*-[5-cyano-1-[(2,4-dimethoxyphenyl)sulphonyl]-3-(2-ethoxyphenyl)-2-oxo-2,3-dihydro-1*H*-indol-3-yl]-4-(4-methylpiperazin-1-yl)piperidine-1-carboxamide (as Example 128).

In addition to the binding affinity to the vasopressin V1 b receptor, further properties may be advantageous in the treatment and/or prophylaxis of vasopressin-dependent disorders, such as, for example:
1.) a selectivity for the vasopressin V1b receptor over the vasopressin V1a receptor, i.e. the quotient of the binding affinity to the V1a receptor (Ki(V1a) (determined in the unit "nanomolar (nM)") and the binding affinity to the V1b receptor (Ki(V1b)) (determined in the unit "nanomolar (nM)"). The greater the quotient Ki(V1a)/Ki(V1b), the greater the V1b selectivity;
2.) a selectivity for the vasopressin V1 b receptor over the vasopressin V2 receptor, i.e. the quotient of the binding affinity to the V2 receptor (Ki(V2) (determined in the unit "nanomolar (nM)") and the binding affinity to the V1 b receptor (Ki(V1 b)) (determined in the unit "nanomolar (nM)"). The greater the quotient Ki(V2)/Ki(V1b), the greater the V1b selectivity;
3.) a selectivity for the vasopressin V1 b receptor over the oxytocin OT receptor, i.e. the quotient of the binding affinity to the OT receptor (Ki(OT) (determined in the unit "nanomolar (nM)") and the binding affinity to the V1b receptor (Ki(V1b)) (determined in the unit "nanomolar (nM)"). The greater the quotient Ki(OT)/Ki(V1b), the greater the V1b selectivity;
4.) the metabolic stability, determined, for example, using the half-life determined in vitro in liver microsomes of various species (for example rat or human);
5.) only minor, if any, inhibition of cytochrom P450 (CYP) enzymes: cytochrom P450 (CYP) is the name for a superfamily of haem proteins having enzymatic activity (oxidases). They are also of particular importance for the degradation (metabolism) of foreign substances, such as pharmaceutics or xenobiotics, in mammalian organisms. The most important representatives of the types and subtypes of CYP in the human organism are: CYP 1A2, CYP 2C9, CYP 2D6 and CYP 3A4. When CYP 3A4 inhibitors (for example grapefruit juice, cimetidine, erythromycin) and medicaments which are degraded via this enzyme system and which thus compete for the same binding site at the enzyme are administered simultaneously, their degradation may be slowed down, and actions and side-effects of the medicament administered may be enhanced in an unwanted manner;
6.) suitable solubility in water (in mg/ml);
7.) suitable pharmacokinetics (temporal profile of the concentration of the compound according to the invention in the plasma or in tissues, for example brain). Pharmacokinetics may be described by the following parameters: half-life, distribution volume, plasma clearance, AUC ("area under the curve", area under the concentration-time curve), oral bioavailability, the brain/plasma ratio;
8.) a certain proportion of the active substance is present attached to plasma proteins (drug/plasma protein binding (PPB) value);
9.) no or only minor blockage of the hERG channel: compounds which block the hERG channel may prolong the QT interval, thus leading to serious irregularities of pulse (for example "torsade de pointes"). Using a displacement assay described in the literature with radioactively labelled dofetilide (G.J. Diaz et al., Journal of Pharmacological and Toxicological Methods, 50 (2004), 187-199), it is possible to determine the potential of compounds of blocking the hERG channels. The lower the IC50 in this "dofetilide assay", the more likely a potent hERG blockage. In addition, the blockage of the hERG channel may be measured by electrophysical experiments using cells transfected with the hERG channel, by "whole-cell patch clamping" (G.J. Diaz et al., Journal of Pharmacological and Toxicological Methods, 50 (2004), 187-199).

It is an object of the present invention to provide a compound with high and selective activity, preferably in particular for the vasopressin V1 b receptor, for the treatment or prophylaxis of various vasopressin-dependent diseases. In addition, the substance according to the invention should have one or more of the advantages 1.) to 9.) mentioned above, in particular a suitable selectivity for the V1b receptor over the V1a receptor.

This object is achieved by the compound of the formula (I) 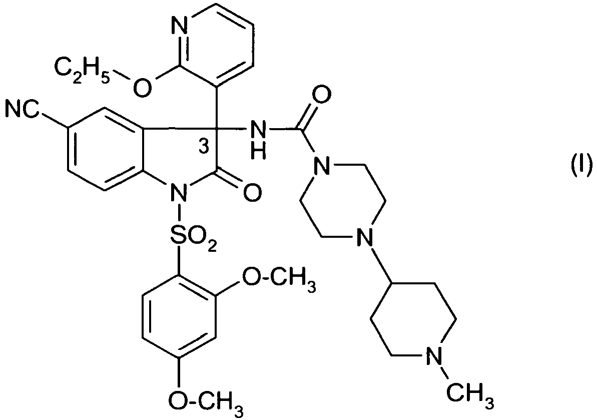 and the pharmaceutically acceptable salts, tautomeric forms and prodrugs thereof.

The compound of the formula (I) of the invention has a centre of chirality in position 3 of the 2-oxindole ring. The compound according to the invention of the formula (I) may therefore be present as a 1:1 mixture of its enantiomers (racemate), or as a non-racemic mixture of its enantiomers in which one of the two enantiomers, i.e. either the (laevorotatory) enantiomer which turns the plane of polarization of linear polarized light to the left ((-)-enantiomer below), or the (dextrorotatory) enantiomer which turns the plane of polarization of linear polarized light to the right ((+)-enantiomer below), is enriched, or as essentially enantiomerically pure compound (enantiomeric excess ee >50 %, in particular > 90%), i.e. as essentially enantiomerically pure (-)-enantiomer or (+)-enantiomer. Preferably, the compounds are present as essentially enantiomerically pure compounds. Particular preference is given to compounds which are essentially enantiomerically pure (ee > 90%).

The invention therefore provides the pure enantiomers of I as well as their mixtures, for example, mixtures in which one enantiomer is present in enriched form, but also the racemates. The invention also provides the pharmaceutically acceptable salts and the tautomers of the pure enantiomers of (I), and the enantiomer mixtures in the form of the pharmaceutically acceptable salts and the tautomers of (I).

A preferred embodiment of the invention relates to the compound of the formula (I) as well as the pharmaceutically acceptable salts and the tautomeric forms thereof, which is characterized in that it is present in optically active form and that it comprises an excess of the enantiomer, which in the form of the free base rotates the plane of polarization of polarized light to the left (i.e. the laevorotatory enantiomer). Below, the laevorotatory enantiomer of the compounds (I) is also referred to as (-)-enantiomer. X-Ray structure analyses have shown that the (-)-enantiomer of the compound of the formula (I) has S configuration with respect to the centre of asymmetry at the carbon atom of position 3 of the oxindole ring. This enantiomer of is also referred to as the enantiomer having the preferred absolute configuration at the C-3 ring carbon atom.

According to the invention, preference is given to the compound of the formula (I), the tautomers thereof and the pharmaceutically acceptable salts thereof as defined above, in which the corresponding (-)-enantiomer is present in an optical purity (enantiomeric excess, ee) of greater than 50%.

According to the invention, preference is given to the compound of the formula (I), the tautomer thereof and the pharmaceutical acceptable salts thereof as defined above, in which the enantiomer having the preferred absolute configuration at the C-3 ring carbon atom is present in an optical purity (enantiomeric excess, ee) of greater than 50%.

According to the invention, preference is given to the compound of the formula (I), the tautomers thereof and the pharmaceutically acceptable salts thereof as defined above, in which the corresponding (-)-enantiomer is present in an optical purity (enantiomeric excess, ee) of greater than 90%.

According to the invention, preference is given to the compound of the formula (I), the tautomer thereof and the pharmaceutical acceptable salts thereof as defined above, in which the enantiomer having the preferred absolute configuration at the C-3 ring carbon atom is present in an optical purity (enantiomeric excess, ee) of greater than 90%.

Likewise preferred embodiments of the invention are the compound of the formula (I) as defined above, which are characterized in that it is present in optically inactive form, that is to say in the form of the racemate, or in the form of a pharmaceutically acceptable salt or a tautomeric form of the racemate.

A further subject-matter of the present invention relates to medicaments comprising the compound of the formula (I) and/or a pharmaceutically acceptable salt thereof as defined above.

A further subject-matter of the present invention relates to the compound of the formula (I) and/or a pharmaceutically acceptable salt thereof as defined above for use as a medicament.

A further subject-matter of the present invention relates to the compound of the formula (I) and/or a pharmaceutically acceptable salt thereof as defined above for use in treatment or prophylaxis of a disease or disorder, in particular a vasopressin-dependent disease or a disease mentioned herein.

A further subject-matter of the present invention relates to the compound of the formula (I) and/or a pharmaceutically acceptable salt thereof as defined above for the treatment and/or prophylaxis of at least one vasopressin-dependent disease and/or for the manufacture of a medicament for the treatment and/or a prophylaxis of at least one vasopressin-dependent disease. Vasopressin-dependent diseases are those in which the progression of the disease depends at least in part on vasopressin, i.e. diseases where the vasopressin level, which may contribute directly or indirectly to the disease picture, is elevated.

The present invention also relates to the compound of the formula (I) and/or a pharmaceutically acceptable salt thereof for the treatment and/or prophylaxis of diseases in which the progression of the disease depends at least in part on vasopressin, i.e. diseases where the vasopressin level, which may contribute directly or indirectly to the disease picture, is elevated. The present invention also relates to the use of the compound of the formula (I) and/or a pharmaceutically acceptable salt thereof for preparing a medicament for the treatment and/or prophylaxis of such a disease.

The present invention relates in particular to the compound of the formula (I) and/or a pharmaceutically acceptable salt thereof as defined above for the treatment and/or prophylaxis of at least one disorder selected from the group consisting of diabetes, in particular diabetes insipidus, insulin resistance, nocturnal enuresis, incontinence, diseases in which blood coagulation disorders occur, and/or for delaying micturition and the use thereof for the manufacture of a medicament for the treatment and/or prophylaxis of at least one of said diseases.

The present invention relates in particular to the compound of the formula (I) and/or a pharmaceutically acceptable salt thereof as defined above for the treatment and/or prophylaxis of at least one disorder selected from the group consisting of hypertension, pulmonary hypertension, heart failure, myocardial infarction, coronary spasm, unstable angina, PTCA (percutaneous transluminal coronary angioplasty), ischemias of the heart, disorders of the renal system, edemas, renal vasospasm, necrosis of the renal cortex, hyponatremia, hypokalemia, Schwartz-Bartter syndrome, disorders of the gastrointestinal tract, gastritic vasospasm, hepatocirrhosis, gastric and intestinal ulcer, emesis, emesis occurring during chemotherapy, and travel sickness, and the use thereof for the manufacture of a medicament for the treatment and/or prophylaxis of at least one of said diseases.

The compound of the formula (I), its salts and its tautomers can also be used for the treatment of various vasopressin-dependent complaints which exhibit central nervous causes or alterations in the HPA axis (hypothalamic pituitary adrenal axis), for example for affective disorders such as depressive disorders and bipolar disorders. These include for example dythymic disorders, phobias, post-traumatic stress disorders, general anxiety disorders, panic disorders, seasonal depressions and sleep disorders.

The compound of the formula (I), its salts and its tautomers can likewise be employed for treatment in cases of anxiety disorders and stress-dependent anxiety disorders such as, for example, generalized anxiety disorders, phobias, post-traumatic anxiety disorders, panic anxiety disorders, obsessive-compulsive anxiety disorders, acute stress-dependent anxiety disorders and social phobia. The compounds of the invention can further be employed also for the treatment of memory impairments, Alzheimer's disease, psychoses, psychotic disorders, sleep disorders and/or Cushing's syndrome, and all stress-dependent diseases.

A further subject-matter of the invention relates to the compound of the formula (I) and/or a pharmaceutically acceptable salt thereof as defined above for the treatment of affective disorders and/or for the manufacture of a medicament for the treatment of affective disorders.

A further subject-matter of the invention relates to the compound of the formula (I) and/or a pharmaceutically acceptable salt thereof as defined above for the treatment of anxiety disorders and/or stress-dependent anxiety disorders and/or for the manufacture of a medicament for the treatment of anxiety disorders and/or stress-dependent anxiety disorders.

A further subject-matter of the invention relates to the compound of the formula (I) and/or a pharmaceutically acceptable salt thereof as defined above for the treatment of memory impairments and/or Alzheimer's disease and/or for the manufacture of a medicament for the treatment of memory impairments and/or Alzheimer's disease.

A further subject-matter of the invention relates to the compound of the formula (I) and/or a pharmaceutically acceptable salt thereof as defined above for the treatment of psychoses and/or psychotic disorders and/or for the manufacture of a medicament for the treatment of psychoses and/or psychotic disorders.

A further subject-matter of the invention relates to the compound of the formula (I) and/or a pharmaceutically acceptable salt thereof as defined above for the treatment of Cushing's syndrome or other stress-dependent diseases and/or for the manufacture of a medicament for the treatment of Cushing's syndrome or other stress-dependent diseases.

A further subject-matter of the invention relates to the compound of the formula (I) and/or a pharmaceutically acceptable salt thereof as defined above for the treatment of sleep disorders and/or for the manufacture of a medicament for the treatment of sleep disorders.

A further subject-matter of the invention relates to the compound of the formula (I) and/or a pharmaceutically acceptable salt thereof as defined above for the treatment of depressive disorders and/or for the manufacture of a medicament for the treatment of depressive disorders.

A further subject-matter of the invention relates to the compound of the formula (I) and/or a pharmaceutically acceptable salt thereof as defined above for the treatment of childhood onset mood disorders and/or for the manufacture of a medicament for the treatment of childhood onset mood disorders. The term "childhood onset mood disorders" is understood to mean mood disorders and depressions which begin as early as childhood.

A further subject-matter of the invention relates to the compound of the formula (I) and/or a pharmaceutically acceptable salt thereof as defined above for the treatment of vasomotor symptoms and/or thermoregulatory dysfunctions, such as, for example, the "hot flush" symptom.

A further subject-matter of the invention relates to the compound of the formula (I) and/or a pharmaceutically acceptable salt thereof as defined above for the treatment and/or prophylaxis of drug dependencies, medicament dependencies and/or dependencies mediated by other factors, for the treatment and/or prophylaxis of stress caused by the withdrawal of one or more factors mediating the dependency and/or for the treatment and/or prophylaxis of stress-induced relapses into the drug dependencies, medicament dependencies and/or dependencies mediated by other factors.

A further subject-matter of the invention relates to the compound of the formula (I) and/or a pharmaceutically acceptable salt thereof as defined above for the treatment and/or prophylaxis of schizophrenia and/or psychosis.

A further subject-matter of the invention relates to compound of formula (I) for use in a method for the treatment and/or prophylaxis of at least one disorder selected from the group consisting of diabetes, in particular diabetes insipidus, insulin resistance, nocturnal enuresis, incontinence, diseases in which blood coagulation disorders occur, and for delaying micturition in a patient, characterized in that an effective amount of the compound of the formula (I) and/or a pharmaceutically acceptable salt thereof is administered to the patient.

A further subject-matter of the invention relates to a compound of formula (I) for use in method for the treatment and/or prophylaxis of at least one disorder selected from the group consisting of hypertension, pulmonary hypertension, heart failure, myocardial infarction, coronary spasm, unstable angina, PTCA (percutaneous transluminal coronary angioplasty), ischemias of the heart, disorders of the renal system, edemas, renal vasospasm, necrosis of the renal cortex, hyponatremia, hypokalemia, Schwartz-Bartter syndrome, disorders of the gastrointestinal tract, gastritic vasospasm, hepatocirrhosis, gastric and intestinal ulcer, emesis, emesis occurring during chemotherapy, and travel sickness in a patient, characterized in that an effective amount of the compound of the formula (I) and/or a pharmaceutically acceptable salt thereof is administered to the patient.

A further subject-matter of the invention relates to a compound of formula (I) for use in method for the treatment and/or prophylaxis of affective disorders in a patient, characterized in that an effective amount of the compound of the formula (I) and/or a pharmaceutically acceptable salt thereof is administered to the patient.

A further subject-matter of the invention relates to a compound of formula (I) for use in method for the treatment of anxiety disorders and/or stress-dependent anxiety disorders in a patient, characterized in that an effective amount of the compound of the formula (I) and/or a pharmaceutically acceptable salt thereof is administered to the patient.

A further subject-matter of the invention relates to a compound of formula (I) for use in method for the treatment of memory impairments and/or Alzheimer's disease in a patient, characterized in that an effective amount of the compound of the formula (I) and/or a pharmaceutically acceptable salt thereof is administered to the patient.

A further subject-matter of the invention relates to a compound of formula (I) for use in a method for the treatment of psychoses and/or psychotic disorders in a patient, characterized in that an effective amount of the compound of the formula (I) or (Ia) and/or a pharmaceutically acceptable salt thereof is administered to the patient.

A further subject-matter of the invention relates to a compound of formula (I) for use in method for the treatment of Cushing's syndrome in a patient, characterized in that an effective amount of the compound of the formula (I) and/or a pharmaceutically acceptable salt thereof is administered to the patient.

A further subject-matter of the invention relates to a compound of formula (I) for use in method for the treatment of sleep disorders in a patient, characterized in that an effective amount of the compound of the formula (I) and/or a pharmaceutically acceptable salt thereof is administered to the patient.

A further subject-matter of the invention relates to a compound of formula (I) for use in method for the treatment of depressive disorders in a patient, characterized in that an effective amount of the compound of the formula (I) and/or a pharmaceutically acceptable salt thereof is administered to the patient.

A further subject-matter of the invention relates to a compound of formula (I) for use in method for the treatment and/or prophylaxis of vasomotor symptoms and/or thermoregulatory dysfunctions, such as, for example, the "hot flush" symptom, in a patient, characterized in that an effective amount of the compound of the formula (I) and/or a pharmaceutically acceptable salt thereof is administered to the patient.

A further subject-matter of the invention relates to a compound of formula (I) for use in method for the treatment and/or prophylaxis of drug dependencies, medicament dependencies and/or dependencies mediated by other factors, for the treatment and/or prophylaxis of stress caused by the withdrawal of one or more factors mediating the dependency and/or for the treatment and/or prophylaxis of stress-induced relapses in the drug dependencies, medicament dependencies and/or dependencies mediated by other factors, in a patient, characterized in that an effective amount of the compound of the formula (I) and/or pharmaceutically acceptable salt thereof is administered to the patient.

A further subject-matter of the invention relates to a compound of formula (I) for use in method for the treatment and/or prophylaxis of schizophrenia and/or psychosis in a patient, characterized in that an effective amount of the compound of the formula (I) and/or a pharmaceutically acceptable salt thereof is administered to the patient.

A further subject-matter of the invention relates to a compound of formula (I) for use in method as defined above, which is characterized in that the patient is a mammal, preferably a human or a nonhuman mammal or a nonhuman transgenic mammal.

The compound of the formula (I), and its pharmaceutically acceptable salts as defined above can be prepared by a skilled worker with knowledge of the technical teaching of the invention in implementation and/or in analogous implementation of process steps known per se.

A further preferred embodiment relates to the compound of the formula (I) and its tautomers, and its pharmaceutically acceptable salts as described above, which are characterized in that they are selective for the vasopressin receptor subtype V1b over at least one of the closely related vasopressin/oxytocin receptor subtypes (for example vasopressin V1a, vasopressin V2 and/or oxytocin).

A further preferred embodiment relates to the compound of the formula (I), its tautomers, and its pharmaceutically acceptable salts as described above, which are characterized in that they have improved metabolic stability.

The metabolic stability of a compound can be determined, for example, by incubating a solution of this compound with liver microsomes from particular species (for example rat, dog or human) and determining the half-life of the compound under these conditions (RS Obach, Curr Opin Drug Discov Devel. 2001, 4, 36-44). It is possible to conclude from an observed larger half-life that the metabolic stability of the compound is improved. The stability in the presence of human liver microsomes is of particular interest since it makes it possible to predict the metabolic degradation of the compound in the human liver. Compounds with increased metabolic stability (determined in the liver microsome test) are therefore probably also degraded more slowly in the liver. The slower metabolic degradation in the liver can lead to higher and/or longer-lasting concentrations (effective levels) of the compound in the body, so that the elimination half-life of the compounds according to the invention is increased. Increased and/or longer-lasting effective levels may lead to a better efficacy of the compound in the treatment or prophylaxis of various vasopressin-dependent diseases. An improved metabolic stability may additionally lead to an increased bioavailability after oral administration, because the compound is subjected, after being absorbed in the intestine, to less metabolic degradation in the liver (so-called first pass effect). An increased oral bioavailability may, because the concentration (effective level) of the compound is increased, lead to a better efficacy of the compound after oral administration.

A further preferred embodiment relates to the compound of the formula (I) as described above, characterized in that, in patients or relevant animal models allowing prognostic statements on therapeutic application, they have improved pharmacological activity compared to the oxindole compounds known from the prior art.

As far as the term "compound" is used in connection with the present invention, it is to be understood as, on the one hand, the racemate of the compound of the formula (I) and the pharmaceutically acceptable salts, tautomeric forms, diastereomeric and/or enantiomeric forms thereof, and, on the other hand, the two enantiomers of the compound of the formula (I), preferably the (-)-enantiomer of the compound of the formula (I) and the pharmaceutically acceptable salts, tautomeric forms thereof.

The invention furthermore relates to the pharmaceutically acceptable salts of the compound of the formula I, which are also referred to as physiologically acceptable salts. The salts are generally obtainable by reacting the free base of the compounds (I) according to the invention with a suitable acid. Suitable acids are listed, for example, in "Fortschritte der Arzneimittelforschung" [Advances in Drug Research], 1966, Birkhäuser Verlag, Vol.10, pp.224-285. They include, for example, hydrochloric acid, citric acid, tartaric acid, lactic acid, phosphoric acid, methanesulphonic acid, acetic acid, formic acid, maleic acid and fumaric acid.

The compounds of the invention are effective after administration by various routes. The administration can, for example, be carried out intravenously, intramuscularly, subcutaneously, topically, intratracheally, intranasally, transdermally, vaginally, rectally, sublingually, buccally or orally and is frequently carried out intravenously, intramuscularly or in particular orally.

The present invention also relates to pharmaceutical compositions which comprise a compound (I) of the invention, and/or a tautomer, and/or a pharmaceutically acceptable salt and/or a prodrug thereof and suitable pharmaceutical carriers (drug carriers). The amount of compound I in the pharmaceutical composition may depend on the formulation type of the composition and may be e.g. in the range from 0.0001 mg/g to 1 g/g in particular from 0.001 mg/g to 0.5 g/g of the composition.

These drug carriers are chosen according to the pharmaceutical form and the desired mode of administration.

The compound of the formula (I) or, where appropriate, suitable salts of this compound can be used to manufacture pharmaceutical compositions for oral, sublingual, buccal, subcutaneous, intramuscular, intravenous, topical, intratracheal, intranasal, transdermal, vaginal or rectal administration and be administered to animals or humans in unit dose forms mixed with conventional pharmaceutical carriers for the prophylaxis or treatment of the above disorders or diseases.

Suitable uniform administration forms (unit dose forms) comprise forms for oral administration, such as tablets, gelatin capsules, powders, granules, solutions or suspensions for oral intake, forms for sublingual, buccal, intratracheal or intranasal administration, aerosols, implants, forms of subcutaneous, intramuscular or intravenous administration and forms of rectal administration.

The compounds of the invention can be used in creams, ointments or lotions for topical administration.

In order to achieve the desired prophylactic or therapeutic effect, the dose of the active compound can vary between 0.01 and 50 mg per kg of body weight and per day.

Each unit dose may comprise from 0.05 to 5000 mg, preferably 1 to 1000 mg, of the active compound in combination with a pharmaceutical carrier. This unit dose can be administered 1 to 5 times a day so that a daily dose of from 0.5 to 25 000 mg, preferably 1 to 5000 mg, is administered.

If a solid composition in the form of tablets is prepared, the active compound is mixed with a pharmaceutical carrier such as gelatin, starch, lactose, magnesium stearate, talc, silicon dioxide or the like.

The tablets can be coated with sucrose, a cellulose derivative or another suitable substance or be treated otherwise in order to display a prolonged or delayed activity and in order to release a predetermined amount of the active compound continuously.

A preparation in the form of gelatin capsules is obtained by mixing the active compound with an extender and taking up the resulting mixture in soft or hard gelatin capsules.

A preparation in the form of a syrup or elixir or for administration in the form of drops may comprise active compounds together with a sweetener which is preferably calorie-free, methylparaben or propylparaben as antiseptics, a flavouring and a suitable colouring.

The water-dispersible powders or granules may comprise the active compounds mixed with dispersants, wetting agents or suspending agents, such as polyvinylpyrrolidones, and sweeteners or taste improvers.

Rectal or vaginal administration is achieved by the use of suppositories which are prepared with binders which melt at the rectal temperature, for example cocoa butter or polyethylene glycols. Parenteral administration is effected by using aqueous suspensions, isotonic salt solutions or sterile and injectable solutions which comprise pharmacologically suitable dispersants and/or wetting agents, for example propylene glycol or polyethylene glycol.

The active compound can also be formulated as microcapsules or centrosomes, if suitable with one or more carriers or additives.

In addition to the compound of the formula (I), or its pharmaceutically acceptable salts the compositions of the invention may comprise further active compounds which may be beneficial for the treatment of the impairments or disorders indicated above.

The present invention therefore further relates to pharmaceutical compositions which comprise a plurality of active compounds, where at least one of these is a compound (I) according to the invention, a tautomer or a salt thereof.

### PREPARATION OF THE COMPOUNDS OF THE INVENTION

An example of a synthetic route for preparing the oxindole derivative of the invention is described below.

Below, the invention is illustrated in more detail using examples, without being limited to the examples mentioned.

### EXPERIMENTAL PART

### EXAMPLE 1

### N-[5-Cyano-1-[(2,4-dimethoxyphenyl)sulphonyl]-3-(2-ethoxypyridin-3-yl)-2-oxo-2,3-dihydro-1H-indol-3-yl]-4-(1-methylpiperidin-4-yl)piperazine-1-carboxamide

### 1a) 3-(2-Ethoxypyridin-3-yl)-3-hydroxy-5-iodo-1,3-dihydro-2H-indol-2-one

With ice-bath cooling, 20.86 g (76.40 mmol) of 5-iodoisatin were stirred in 400 ml of anhydrous tetrahydrofuran (THF), and 3.22 g (80.50 mmol, 60% w/w) of sodium hydride were added a little at a time, the temperature being kept between 0-10°C. With ice-bath cooling, the suspension was stirred for one hour, during which the pyridine Grignard reagent was prepared. At room temperature, 20 g (80.30 mmol) of the 2-ethoxy-3-iodopyridine were dissolved in 400 ml of anhydrous THF, and over a period of 5-10 minutes 95.6 ml (1M solution in THF, 95.60 mmol) of ethylmagnesium bromide were added to this solution with cooling, at a temperature between 22 and 15°C. The solution was stirred for 20 minutes, during which time the colour changed from colourless to slightly yellowish.

The solution of the pyridine Grignard reagents was then, over a period of 5-10 minutes, added to the solution, cooled in an ice-bath, of the 5-iodoisatin sodium salt, the temperature fluctuating between 5 and 18°C. After the addition of the Grignard reagent had ended, the ice-bath was removed, and the reaction mixture was stirred at room temperature for another 2 hours. Excess saturated ammonium chloride solution was added, followed by ethyl acetate, and the mixture was stirred for another 5 minutes. The aqueous phase was removed and extracted with ethyl acetate (2 x). The combined organic phases were washed with water (2 x), and the solvent was removed under reduced pressure. Initially, unreacted 5-iodoisatin precipitated from the still dilute solution and was removed, and after further concentration the product, too, crystallized out. The suspension was stored in a refrigerator at 5°C for two hours and the slightly yellowish solid was then filtered off and washed with a little ethyl acetate. The desired 3-(2-ethoxypyridin-3-yl)-3-hydroxy-5-iodo-1,3-dihydro-2*H*-indol-2-one (17.1 g, 43.16 mmol, 57%) was isolated after drying at 40°C.
ESI-MS [M+H⁺] = 397.05 Calculated for C₁₅H₁₃IN₂O₃ = 396.19

### 1b) 5-Cyano-3-hydroxy-3-(2-ethoxypyridin-3-yl)-1,3-dihydroindol-2-one

Under an atmosphere of nitrogen, 7.1 g (17.92 mmol) of 3-(2-ethoxypyridin-3-yl)-3-hydroxy-5-iodo-1,3-dihydro-2H-indol-2-one were stirred in 100 ml of anhydrous THF at room temperature. 2.1 g (17.92 mmol) of zinc cyanide were added, followed by 0.51 g (0.45 mmol) of tetrakis(triphenylphosphine)palladium(0). The reaction mixture was transferred directly into a preheated oil bath at a temperature of 100°C. The mixture was stirred at 100°C (oil bath temperature), and after 30 minutes, another 0.51 g (0.45 mmol) of the catalyst was added. In total, the mixture was stirred for 2 hours. The reaction mixture was cooled to room temperature, and an excess of water was added. The mixture was extracted with ethyl acetate (3 x), and the combined organic phases were washed with water (3 x). The solvent was evaporated to dryness under reduced pressure, and the residue was slurried with small volumes of ethyl acetate. A slightly yellowish solid was removed by filtration, washed with ethyl acetate and dried in a vacuum drying cabinet. It was possible to isolate 3.7 g (12.44 mmol, 69.4%) of the desired product 5-cyano-3-hydroxy-3-(2-ethoxypyridin-3-yl)-1,3-dihydroindol-2-one.
ESI-MS [M+H⁺] = 296.05 Calculated for C₁₆H₁₃N₃O₃ = 295.30

### 1c) 3-Chloro-3-(2-ethoxypyridin-3-yl)-2-oxoindoline-5-carbonitrile

Under an atmosphere of nitrogen, 6.00 g (20.32 mmol) of the 5-cyano-3-hydroxy-3-(2-ethoxypyridin-3-yl)-1,3-dihydroindol-2-one were suspended in 60 ml of anhydrous dichloromethane (dried over molecular sieve). 2.30 ml (28.45 mmol) of pyridine were then added. The reaction mixture was cooled to a temperature of 0°C, and 2.06 ml (28.45 mmol) of neat thionyl chloride were then added dropwise (exothermic reaction). The mixture was stirred at room temperature for one hour. The formation of a yellow suspension was observed. The course of the reaction was monitored by thin-layer chromatography (TLC) (silica gel, dichloromethane/methanol in a ratio of 95:5). The reaction mixture was carefully poured into ice-water. After 15 minutes of stirring, the organic phase was removed. The aqueous phase was extracted with dichloromethane (2 x). All organic phases were combined, dried over magnesium sulphate and filtered, and the solvent was removed under reduced pressure. The product gave 5.70 g (18.17 mmol, 89%) of 3-chloro-3-(2-ethoxypyridin-3-yl)-2-oxoindoline-5-carbonitrile as an amorphous solid which was used without further purification for the next reaction.
ESI-MS [M+H⁺] = 314.1 Calculated for C₁₆Hₗ₂CIN₃O₂ = 313.75

### 1 d) 3-Amino-3-(2-ethoxypyridin-3-yl)-2-oxoindoline-5-carbonitrile

5.70 g (18.17 mmol) of 3-chloro-3-(2-ethoxypyridin-3-yl)-2-oxoindoline-5-carbonitrile were dissolved in 50 ml of dichloromethane. Under an atmosphere of nitrogen, 14 ml (98.11 mmol) of a 7 *N* methanolic ammonia solution were slowly added dropwise to the cooled reaction solution. The colour of the solution changed to light-yellow, and the solution was stirred at room temperature overnight, during which time the product slowly crystallized out. The course of the reaction was monitored by TLC (silica gel, dichloromethane/methanol in a ratio of 9:1). The solvent was removed under reduced pressure, and the residue was once more taken up and dissolved in dichloromethane. The mixture was then extracted with water. The phases were separated, and a greasy phase which had formed between the phases was added to the aqueous phase. The aqueous phase was extracted with ethyl acetate until the greasy phase had gone into solution. All organic phases obtained were combined, and the solvent was removed under reduced pressure. The residue was triturated with diethyl ether, resulting in the formation of a solid substance which was filtered off and dried in a vacuum drying cabinet at moderate temperature (35°C). This gave 4.54 g (15.43 mmol, 85%) of the 3-amino-3-(2-ethoxypyridin-3-yl)-2-oxoindoline-5-carbonitrile as a solid.
ESI-MS [M+H⁺] = 295.3 Calculated for C₁₆H₁₄N₄O₂ = 294.32

### 1e) 3-Amino-1-[(2,4-dimethoxyphenyl)sulphonyl]-3-(2-ethoxypyridin-3-yl)-2-oxoindoline-5-carbonitrile

3.54 g (12.03 mmol) of 3-amino-3-(2-ethoxypyridin-3-yl)-2-oxoindoline-5-carbonitrile were dissolved in 80 ml of anhydrous dimethylformamide (dried over molecular sieve). Under an atmosphere of nitrogen and with cooling using an ice-bath, 1.49 g (13.23 mmol) of potassium *tert*-butoxide were added a little at a time. The colour of the reaction mixture changed, and the brown solution was stirred at 0°C for another hour to ensure that the deprotonation proceeded to completion. At low temperature, 3.16 g (13.23 mmol) 2,4-dimethoxybenzenesulphonyl chloride were added, and the mixture was stirred at 0°C for another two hours. The course of the reaction was monitored by TLC (silica gel, dichloromethane/methanol in a ratio of 9:1). The reaction mixture was poured into ice-water and then extracted with ethyl acetate. The organic phase was washed with saturated sodium chloride solution and dried over magnesium sulphate, and the solvent was evaporated. The residue was suspended in diethyl ether and stirred until the product precipitated as a solid and could be removed by filtration. After removal of the solvent, the mother liquor was once more treated with diethyl ether (2 x) until finally, after drying, 4.67 g (9.44 mmol, 79%) of the desired 3-amino-1-[(2,4-dimethoxyphenyl)sulphonyl]-3-(2-ethoxypyridin-3-yl)-2-oxoindoline-5-carbonitrile were obtained as a solid substance.
ESI-MS [M+H⁺] = 495.15 Calculated for C₂₄H₂₂N₄O₆S = 494.53

### 1f) Phenyl [5-cyano-1-[(2,4-dimethoxyphenyl)sulphonyl]-3-(2-ethoxypyridin-3-yl)-2-oxo-2,3-dihydro-1H-indo/-3-yl]carbamate

4.67 g (9.44 mmol) of 3-amino-1-[(2,4-dimethoxyphenyl)sulphonyl]-3-(2-ethoxypyridin-3-yl)-2-oxoindoline-5-carbonitrile were dissolved in 120 ml of pyridine and cooled to 0°C using an ice-bath. 1.30 ml (10.39 mmol) of neat phenyl chloroformate were added, and the reaction mixture was stirred at 0°C for 2 hours. The course of the reaction was monitored by TLC (silica gel, dichloromethane/methanol in a ratio of 95:5). The solvent and especially pyridine were removed under reduced pressure, and the residue was diluted with water and extracted with ethyl acetate (3 x). The combined organic phases were washed with saturated sodium chloride solution, dried over magnesium sulphate and filtered, and the solvent was removed under reduced pressure. Traces of pyridine were removed by repeated addition of toluene and evaporation on a rotary evaporator. Diethyl ether was added to the isolated residue, and a solid crystallized overnight giving 5.62 g (9.14 mmol, 97%) of the desired product phenyl [5-cyano-1-[(2,4-dimethoxyphenyl)sulphonyl]-3-(2-ethoxypyridin-3-yl)-2-oxo-2,3-dihydro-1*H*-indole-3-yl]carbamate.
ESI-MS [M+H⁺] = 615.15 Calculated for C₃₁H₂₆N₄O₈S = 614.64

### 1g) N-[5-Cyano-1-[(2,4-dimethoxyphenyl)sulphonyl]-3-(2-ethoxypyridin-3-yl)-2-oxo-2,3-dihydro-1H-indol-3-yl]-4-(1-methylpiperidin-4-yl)piperazine-1-carboxamide

1.00 g (1.63 mmol) of phenyl [5-cyano-1-[(2,4-dimethoxyphenyl)sulphonyl]-3-(2-ethoxypyridin-3-yl)-2-oxo-2,3-dihydro-1*H*-indol-3-yl]carbamate, 596 mg (3.25 mmol) of 1-(1-methylpiperidin-4-yl)piperazine and 8 ml of dried THF were combined and the mixture was stirred at room temperature for 24 hours. The end of the reaction was detected with the aid of analytical HPLC (RP, eluents acetonitrile/water, 0.01% TFA). The solvent was removed, and the residue was purified by preparative HPLC using dichloromethane and 6% methanol as eluents on a Chromolith column (normal phase, from Merck). After repeated column chromatography, it was possible to isolate 230 mg (0.33 mmol, 21%) of the *N*-[5-cyano-1-[(2,4-dimethoxyphenyl)sulphonyl]-3-(2-ethoxypyridin-3-yl)-2-oxo-2,3-dihydro-1*H*-indol-3-yl]-4-(1-methylpiperidin-4-yl)piperazine-1-carboxamide.

Alternatively, work-up and purification after the reaction had ended could be carried out as follows: the solvent was removed. The crude material was dissolved in ethyl acetate and extracted with 1 *N* HCl. The impurities were detected in the organic phase, the product being in the acidic aqueous phase. Accordingly, the aqueous phase was neutralized with 2 N NaOH solution and extracted with ethyl acetate. After drying over magnesium sulphate, filtration and removal of the ethyl acetate under reduced pressure, the product could be crystallized with diethyl ether. The *N*-[5-cyano-1-[(2,4-dimethoxyphenyl)sulphonyl]-3-(2-ethoxypyridin-3-yl)-2-oxo-2,3-dihydro-1*H*-indol-3-yl]-4-(1-methylpiperidin-4-yl)piperazine-1-carboxamide was obtained in yields of > 50%.
ESI-MS [M+H⁺] = 704.2 Calculated for C₃₅H₄₁N₇O₇S = 703.82
¹H-NMR ([D6]-DMSO, 400 MHz) δ [ppm] = 8.12 (d, 1H, J = 4.8 Hz), 7.88 (d, 1H, J = 8.8 Hz), 7.87 (d, 1H, J = 8.7 Hz), 7.81 (d, 1H, J = 8.5 Hz), 7.72 (d, 1H, J = 7.6 Hz), 7.67 (s, 1H), 7.64 (s, 1H), 7.02 (dd, 1H, J = 5.0 Hz, J = 7.5 Hz), 6.69 (d, 1H, J = 8.9 Hz), 6.65 (s, 1H), 4.15 (m, 2H), 3.85 (s, 3H), 3.44 (s, 3H), 3.20 (m, 4H), 2.76 (m, 2H, J = 11.1 Hz), 2.34 (m, 4H), 2.11 (m, 4H), 1.81 (m, 2H, J = 11.3 Hz), 1.64 (m, 2H, J = 10.7 Hz), 1.37 (m, 2H), 1.06 (t, 1H, J = 7.0 Hz).

RACEMATE RESOLUTION of the racemic compound according to EXAMPLE 1:
The separation of the racemate according to EXAMPLE 1 into its enantiomers (Examples 1 A and 1 B) by separation on a preparative chiral column is described below:
   A.) RACEMATE RESOLUTION of the racemic compound according to EXAMPLE 1: 100 mg (0.14 mmol) of the racemic *N*-[5-cyano-1-[(2,4-dimethoxyphenyl)sulphonyl]-3-(2-ethoxypyridin-3-yl)-2-oxo-2,3-dihydro-1*H*-indol-3-yl]-4-(1-methylpiperidin-4-yl)piperazine-1-carboxamide (EXAMPLE 1) were separated on a chiral preparative column (Chiralcell OD, flow rate 55 ml/min) using *n*-heptane/ethanol (700:300) as eluent. The enantiomer which eluted first, having a positive optical rotation (Example 1A), could be isolated in a yield of 19 mg (0.03 mmol, 19%) and the enantiomer which followed, having a negative optical rotation (Example 1 B), could be isolated in a yield of 8 mg (0.01 mmol, 8%).

### EXAMPLE 1A:

### (+)-N-[5-Cyano-1-[(2,4-dimethoxyphenyl)sulphonyl]-3-(2-ethoxypyridin-3-yl)-2-oxo-2,3-dihydro-1H-indol-3-yl]-4-(1-methylpiperidin-4-yl)piperazine-1-carboxamide

ESI-MS [M+H⁺] = 704.25 Calculated for C₃₅H₄₁N₇O₇S = 703.82
HPLC (Chiralcel OD 0.46 cm x 25 cm; n-heptane/ethanol 7:3) R_{f} = 9.04 min
Optical rotation α (22°C, 589 nm, CHCl₃, 1 mg/ml) = dextrorotatory
¹H-NMR ([D6]-DMSO, 500 MHz) δ [ppm] = 8.13 (dd, 1H, J = 1.6 Hz, J = 4.9 Hz), 7.89 (d, 1H, J = 8.9 Hz), 7.88 (d, 1H, J = 8.6 Hz), 7.82 (dd, 1H, J = 1.7 Hz, J = 8.6 Hz), 7.72 (dd, 1H, J = 1.5 Hz, J = 7.7Hz), 7.68 (d, 1H, J = 1.6 Hz), 7.65 (s, 1H), 7.02 (dd, 1H, J = 4.9 Hz, J = 7.6 Hz), 6.69 (dd, 1H, J = 2.2 Hz, J = 8.9 Hz), 6.66 (d, 1H, J = 2.1 Hz), 4.17 (m, 2H), 3.86 (s, 3H), 3.45 (s, 3H), 3.21 (m, 4H), 2.77 (m, 2H, J = 11.0 Hz), 2.34 (m, 4H), 2.12 (m, 4H), 1.82 (m, 2H, J = 10.9 Hz), 1.64 (m, 2H, J = 10.8 Hz), 1.37 (m, 2H), 1.08 (t, 3H, J = 7.0 Hz).

### EXAMPLE 1B:

### (-)-N-[5-Cyano-1-[(2,4-dimethoxyphenyl)sulphonyl]-3-(2-ethoxypyridin-3-yl)-2-oxo-2,3-dihydro-1H-indol-3-yl]-4-(1-methylpiperidin-4-yl)piperazine-1-carboxamide

ESI-MS [M+H⁺] = 704.25 Calculated for C₃₅H₄₁N₇O₇S = 703.82
HPLC (Chiralcel OD 0.46 cm x 25 cm; n-heptane/ethanol 7:3) R_{f} = 25.73 min
Optical rotation α (22°C, 589 nm, CHCl₃, 1 mg/ml) = laevorotatory
¹H-NMR ([D6]-DMSO, 500 MHz) δ [ppm] = 8.13 (dd, 1H, J = 1.2 Hz, J = 4.7 Hz), 7.88 (d, 1H, J = 8.9 Hz), 7.87 (d, 1H, J = 8.5 Hz), 7.81 (dd, 1H, J = 1.5 Hz, J = 8.5 Hz), 7.72 (dd, 1H, J = 1.1 Hz, J = 7.6 Hz), 7.68 (s, 1H), 7.64 (s, 1H), 7.01 (dd, 1H, J = 4.9 Hz, J = 7.6 Hz), 6.69 (dd, 1H, J = 1.9 Hz, J = 9.0 Hz), 6.66 (d, 1H, J = 1.9 Hz), 4.16 (m, 2H), 3.85 (s, 3H), 3.45 (s, 3H), 3.20 (m, 4H), 2.77 (m, 2H, J = 11.5 Hz), 2.34 (m, 4H), 2.12 (m, 4H), 1.82 (m, 2H, J = 11.3 Hz), 1.64 (m, 2H, J = 11.5 Hz), 1.37 (m, 2H), 1.07 (t, 3H, J = 7.0 Hz).

### METHODS FOR DETERMINING THE BIOLOGICAL ACTIVITY

### Vasopressin V1 b receptor binding assay:

### Substances:

The test substances were dissolved in a concentration of 10⁻² M in DMSO and further diluted to 5x10⁻⁴ M to 5x10⁻⁹ M in DMSO. This series of DMSO predilutions was diluted 1:10 with assay buffer. The substance concentration was again diluted 1:5 in the assay mixture (2% DMSO in the mixture).

### Membrane preparation:

CHO-K1 cells with stably expressed human vasopressin V1b receptor (clone 3H2) were harvested and homogenized in 50 mM Tris-HCl and in the presence of protease inhibitors (Roche complete Mini # 1836170) with a Polytron homogenizer at a medium setting for 2x10 seconds and subsequently centrifuged at 40 000 x g for 1 h. The membrane pellet was again homogenized and centrifuged as described and then taken up in 50 mM Tris-HCl, pH 7.4, homogenized and stored in aliquots frozen in liquid nitrogen at -190°C.

### Binding assay:

The binding assay was carried out by a method based on that of Tahara et al. (Tahara A et al., Brit. J. Pharmacol. 125, 1463-1470 (1998)). The incubation buffer was: 50 mM Tris, 10 mM MgCl₂, 0.1% BSA, pH 7.4.

In the assay mixture (250 µl), membranes (50 µg/ml protein in incubation buffer) from CHO-K1 cells with stably expressed human V1b receptors (cell line hV1b_3H2_CHO) were incubated with 1.5 nM ³H-AVP (8-Arg-vasopressin, PerkinElmer #18479) in incubation buffer (50 mM Tris, 10 mM MgCl₂, 0.1% BSA, pH 7.4) (total binding) or additionally with increasing concentrations of test substance (displacement experiment). The nonspecific binding was determined with 1 µM AVP (Bachem # H1780). All determinations were carried out as triplicate determinations. After incubation (60 minutes at room temperature), the free radioligand was removed by vacuum filtration (Skatron cell harvester 7000) through Wathman GF/B glass fibre filter mats, and the filters were transferred into scintillation vials. The liquid scintillation measurement took place in a Tricarb model 2000 or 2200CA instrument (Packard). Conversion of the measured cpm into dpm was carried out with the aid of a standard quench series.

### Evaluation:

The binding parameters were calculated by nonlinear regression in SAS. The algorithms of the program operate in analogy to the LIGAND analysis program (Munson PJ and Rodbard D, Analytical Biochem. 107, 220-239 (1980)). The Kd of ³H-AVP for the recombinant human V1b receptors is 0.4 nM and was used to determine the Ki value.

The test reveals that compound of examples 1 and 1 B of the present invention have a high affinity towards the V1b receptor which, expressed as Kᵢ(h-V1b) values, is ast most 10 nM.

### Vasopressin V1a receptor binding assay:

### Substances:

The test substances were dissolved in a concentration of 10⁻² M in DMSO. These DMSO solutions were further diluted in incubation buffer (50 mM Tris, 10 mM MgCl₂, 0.1% BSA, pH 7.4).

### Membrane preparation:

CHO-K1 cells with stably expressed human vasopressin V1a receptor (clone 5) were harvested and homogenized in 50 mM Tris-HCl and in the presence of protease inhibitors (Roche complete Mini # 1836170) with a Polytron homogenizer at a medium setting for 2x10 seconds and subsequently centrifuged at 40 000 x g for 1 h. The membrane pellet was again homogenized and centrifuged as described and then taken up in 50 mM Tris-HCl, pH 7.4, homogenized and stored in aliquots frozen in liquid nitrogen at -190°C.

### Binding assay:

The binding assay was carried out by a method based on that of Tahara et al. (Tahara A et al., Brit. J. Pharmacol. 125, 1463-1470 (1998)).

The incubation buffer was: 50 mM Tris, 10 mM MgCl₂, 0.1 % BSA, pH 7.4.

In the assay mixture (250 µl), membranes (20 µg/ml protein in incubation buffer) from CHO-K1 cells with stably expressed human V1a receptors (cell line hV1a_5_CHO) were incubated with 0.04 nM ¹²⁵I-AVP (8-Arg-vasopressin, NEX 128) in incubation buffer (50 mM Tris, 10 mM MgCl₂, 0.1% BSA, pH 7.4) (total binding) or additionally with increasing concentrations of test substance (displacement experiment). The nonspecific binding was determined with 1 µM AVP (Bachem # H1780). Triplicate determinations were carried out. After incubation (60 minutes at room temperature), the free radioligand was removed by vacuum filtration (Skatron cell harvester 7000) through Wathman. GF/B glass fibre filter mats, and the filters were transferred into scintillation vials.

The liquid scintillation measurement took place in a Tricarb model 2000 or 2200CA instrument (Packard). Conversion of the measured cpm into dpm was carried out with the aid of a standard quench series.

### Evaluation:

The binding parameters were calculated by nonlinear regression in SAS. The algorithms of the program operate in analogy to the LIGAND analysis program (Munson PJ and Rodbard D, Analytical Biochem. 107, 220-239 (1980)). The Kd of ¹²⁵I-AVP for the recombinant hV1a receptors was determined in saturation experiments. A Kd of 1.33 nM was used to determine the Ki value.

The test reveals that compounds of Examples 1 and 1 B have selectivity towards the V1 b receptor in comparison with V1a receptor, which, expressed as Kᵢ(h-V1a)/Kᵢ(h-V1b) values exceeds 100.

### Vasopressin V2 receptor binding assay:

### Substances:

The test substances were dissolved in a concentration of 10⁻² M in DMSO. This DMSO solution was further diluted in incubation buffer (50 mM Tris, 10 mM MgCl₂, 0.1 % BSA, pH 7.4).

### Membrane preparation:

CHO-K1 cells with stably expressed human vasopressin V2 receptor (clone 23) were harvested and homogenized in 50 mM Tris-HCl and in the presence of protease inhibitors (Roche complete Mini # 1836170) with a Polytron homogenizer at a medium setting for 2x10 seconds and subsequently centrifuged at 40 000 x g for 1 h. The membrane pellet was again homogenized and centrifuged as described and then taken up in 50 mM Tris-HCl, pH 7.4, homogenized and stored in aliquots frozen in liquid nitrogen at -190°C.

### Binding assay:

The binding assay was carried out by a method based on that of Tahara et al. (Tahara A et al., Brit. J. Pharmacol. 125, 1463-1470 (1998)).

The incubation buffer was: 50 mM Tris, 10 mM MgCl₂, 0.1% BSA, pH 7.4.

In the assay mixture (250 µl), membranes (50 µg/ml protein in incubation buffer) from CHO-K1 cells with stably expressed human V2 receptors (cell line hV2_23_CHO) were incubated with 1-2 nM ³H-AVP (8-Arg-vasopressin, PerkinElmer #18479) in incubation buffer (50 mM Tris, 10 mM MgCl₂, 0.1% BSA, pH 7.4) (total binding) or additionally with increasing concentrations of test substance (displacement experiment). The nonspecific binding was determined with 1 µM AVP (Bachem # H1780). Triplicate determinations were carried out.

After incubation (60 minutes at room temperature), the free radioligand was removed by vacuum filtration (Skatron cell harvester 7000) through Wathman GF/B glass fibre filter mats, and the filters were transferred into scintillation vials.

The liquid scintillation measurement took place in a Tricarb model 2000 or 2200CA instrument (Packard). Conversion of the measured cpm into dpm was carried out with the aid of a standard quench series.

### Evaluation:

The binding parameters were calculated by nonlinear regression in SAS. The algorithms of the program operate in analogy to the LIGAND analysis program (Munson PJ and Rodbard D, Analytical Biochem. 107, 220-239 (1980)). The Kd of ³H-AVP for the recombinant hV2 receptors is 2.4 nM and was used to determine the Ki value.

The test reveals that compounds of Examples 1 and 1 B have selectivity towards the V1 b receptor in comparison with V2 receptor, which, expressed as Kᵢ(h-V2)/Kᵢ(h-V1b) values exceeds 50.

### Oxytocin receptor binding assay

### Substances:

The substances were dissolved in a concentration of 10⁻² M in DMSO and diluted with incubation buffer (50 mM Tris, 10 mM MgCl₂, 0.1 % BSA, pH 7.4).

### Cell preparation:

Confluent HEK-293 cells with transiently expressing recombinant human oxytocin receptors were centrifuged at 750 x g and at room temperature for 5 minutes. The residue was taken up in ice-cold lysis buffer (50 mM Tris-HCl, 10% glycerol, pH 7.4 and Roche Complete Protease Inhibitor) and subjected to an osmotic shock at 4°C for 20 minutes. The lyzed cells were then centrifuged at 750 x g and at 4°C for 20 minutes, the residue was taken up in incubation buffer, and aliquots of 10⁷ cells/ml were prepared. The aliquots were frozen at -80°C until used.

### Binding assay:

On the day of the experiment, the cells were thawed, diluted with incubation buffer and homogenized using a Multipette Combitip (Eppendorf, Hamburg). The reaction mixture of 0.250 ml was composed of 2 to 5x10⁴ recombinant cells, 3-4 nM ³H-oxytocin (PerkinElmer, NET 858) in the presence of test substance (inhibition plot) or only incubation buffer (total binding). The nonspecific binding was determined with 10⁻⁶ M oxytocin (Bachem AG, H2510). Determinations in triplicate were set up. Bound and free radioligand were separated by filtration under vacuum with Whatman GF/B glass fibre filters using a Skatron cell harvester 7000. The bound radioactivity was determined by liquid scintillation measurement in a Tricarb beta counter, model 2000 or 2200CA (Packard).

### Evaluation:

The binding parameters were calculated by nonlinear regression analysis (SAS), in analogy to the LIGAND program of Munson and Rodbard (Analytical Biochem 1980; 107: 220-239). The Kd of ³H-oxytocin for the recombinant hOT receptors is 7.6 nM and was used to determine the Ki value.

The test reveals that compounds of Examples 1 and 1 B have selectivity towards the V1 b receptor in comparison with OT receptor, which, expressed as Kᵢ(h-OT)/Kᵢ(h-V1b) values exceeds 100.

### Determination of the microsomal half-life:

The metabolic stability of the compounds of the invention was determined in the following assay.

The test substances are incubated in a concentration of 0.5 µM as follows:
0.5 µM test substance is preincubated together with liver microsomes of various species (rat, human or other species) (0.25 mg of microsomal protein/ml) in 0.05M potassium phosphate buffer pH 7.4 in microtitre plates at 37°C for 5 min. The reaction is started by adding NADPH (1 mg/ml). 50 µl aliquots are taken after 0, 5, 10, 15, 20 and 30 min, and the reaction is stopped immediately with the same volume of acetonitrile and cooled down. The samples are frozen until analyzed. Using MSMS, the remaining concentration of undegraded test substance is determined. From the increase of the test substance signal/time unit curve, the half-life (T1/2) is determined, where the half-life of the test substance can be calculated, assuming first order kinetics, from the decrease in the concentration of the compound with time. The microsomal clearance (mCl) is calculated as mCl= In2/T1/2 / (content of microsomal protein in mg/ml) x 1000 [ml/min/mg] (modified according to the literature references: Di, The Society for Biomoleculaur Screening, 2003, 453-462; Obach, DMD, 1999 vol 27. N 11, 1350-1359).

The test reveals that compounds of Examples 1 and 1 B have a high metabolic stability, which result in human microsomal clearance values of generally at most 60 µl min⁻¹ mg⁻¹.

### Determination of plasma protein binding (PPB) by equilibrium dialysis:

150 µl of rat or human plasma, with 1 or 10 µM of test substance added, is pipetted onto one side of the 96-well dialysis chambers, 150 µl of PPS buffer are pipetted onto the other side. The chambers are separated by a dialysis membrane having a cut-off of 6-8000 dalton.

The 96-well dialysis chambers are covered and gently shaken overnight.

The next morning, 10 µl of plasma are removed and diluted with 90 µl of PPS buffer, and the protein is precipitated using 200 µl of acetonitrile. The precipitated protein is removed by centrifugation, and 100 µl of the supernatant are used for MSMS analysis. From the buffer side, 100 µl are removed for MSMS analysis. See also the following literature reference: Banker, Journal of Pharmaceutical Sciences Vol. 92, 5, 967-974, 2003.

### Methods for the in vitro determination of cytochrom P450 (CYP) inhibition

### Luminescence substrates for 2C9 and 3A4:

0.4 mg/ml of human liver microsomes are preincubated for 10 minutes with the test substances to be tested (0-20 µM), the CYP specific substrates, in 0.05 M potassium phosphate buffer pH 7.4 at 37°C. The Cyp-specific substrate for CYP 2C9 is luciferin H, that for CYP 3A4 is luciferin BE. The reaction is started by addition of NADPH. After 30 min of incubation at RT, the luciferin detection reagent is added, and the resulting luminescence signal is measured (modified according to literature reference: Promega, Technical Bulletin P450-GLO™ Assays).

### Midazolam CYP 3A4 time-dependent inhibition

The test consists of 2 parts. In the first part, the test substance is preincubated with the liver microsomes (with NADPH)= preincubation, followed by addition of the substrate; in the second part, substrate and test substance are added simultaneously = coincubation.

### Preincubation:

0.05 mg/ml of microsomal protein (human liver microsomes) are preincubated with 0-10 µM (or 50 µM) of test substance in 50 mM potassium phosphate buffer for 5 min. The reaction is started using NADPH. After 30 min, 4 µM of midazolam (final concentration) are added, and the mixture is incubated for a further 10 min. After 10 min, 75 µl of the reaction solution are removed and quenched with 150 µl of acetonitrile solution.

### Coincubation:

0.05 mg/ml of microsomal protein (human liver microsomes), 4 µM midazolam (final concentration) and 0-10 µM (or 50 µM) of test substance are preincubated in 50 mM potassium phosphate buffer for 5 min. The reaction is started using NADPH. After 10 min, 75 µl of the reaction solution are removed and quenched with 150 µl of acetonitrile solution. The samples are frozen until analyzed by MSMS (modified according to literature references:
Obdach, Journal of Pharmacology & Experimental Therapeutics, Vol 316, 1, 336-348, 2006; Walsky, Drug Metabolism and Disposition Vol 32, 6, 647-660, 2004).

### Method for determining the solubility in water (in mg/ml)

The solubility in water of the compounds according to the invention can be determined, for example, according to the so-called "shake flask" method (according to ASTM International: E 1148-02, Standard test methods for measurement of aqueous solubility, Book of Standards Volume 11.05.*).* Here, an excess of the solid compound is added to a buffer solution having a certain pH (for example phosphate buffer pH 7.4) and the resulting mixture is shaken or stirred until the steady state has been reached (typically 24 or 48 hours, sometimes even up to 7 days). The undissolved solid is then removed by filtration or centrifugation, and the concentration of the dissolved compound is determined by UV spectroscopy or high-pressure liquid chromatography (HPLC) using an appropriate calibration curve.

## Claims

1. Compound of the general formula (I) 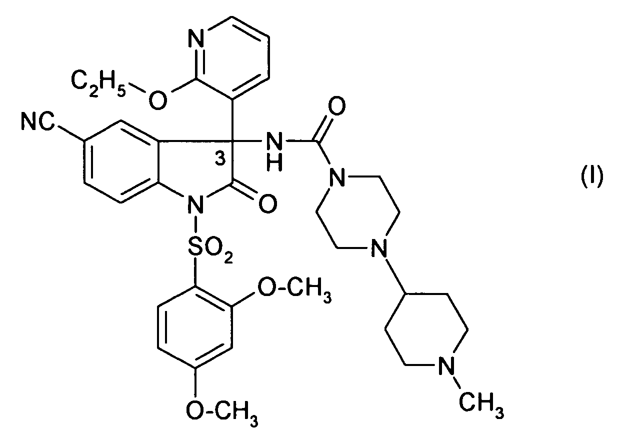 and the pharmaceutically acceptable salts and tautomeric forms thereof.

2. The compound of the formula (I) according to Claim 1, **characterized in that** it is present in optically active form and that it comprises an excess of the enantiomer, which in the form of the free base rotates the plane of polarization of linear polarized light to the left,
and the pharmaceutically acceptable salts and tautomeric forms thereof.

3. The compound of the formula (I) according to Claim 1, **characterized in that** it is present in optically active form, and that it comprises an excess of the enantiomer, wherein the absolute configuration of the chiral C-3 ring carbon atom is S configuration.

4. The compound of the formula (I) according to Claim 2 in optically active form, **characterized in that** the corresponding laevorotatory (-)-enantiomer is present in an optical purity (enantiomeric excess, ee) of greater than 50%.

5. The compound of the formula (I) according to Claim 3 in optically active form, **characterized in that** the enantiomer having the preferred absolute configuration at the C-3 ring carbon atom is present in an optical purity (enantiomeric excess, ee) of greater than 50%.

6. The compound of the formula (I) according to Claim 2 in optically active form, **characterized in that** the corresponding laevorotatory (-)-enantiomer is present in an optical purity (enantiomeric excess, ee) of greater than 90%.

7. The compound of the formula (I) according to Claim **3** in optically active form, **characterized in that** the enantiomer having the preferred absolute configuration at the C-3 ring carbon atom is present in an optical purity (enantiomeric excess, ee) of greater than 90%.

8. Medicament, comprising a compound of the formula (I) according to any of Claims **1 to 7** or at least one pharmaceutically acceptable salt or one tautomeric form thereof.

9. A compound of the formula (I) according to any of Claims **1 to 7,** or a pharmaceutically acceptable salt or a tautomeric form thereof for use as a medicament.

10. Use of the compound of the formula (I) according to any of Claims **1 to 7** or at least one pharmaceutically acceptable salt or one tautomeric form thereof for the manufacture of a medicament for the treatment and/or prophylaxis of a disease or disorder.

11. Use of the compound of the formula (I) according to any of Claims **1 to 7** or at least one pharmaceutically acceptable salt or one tautomeric form thereof for the manufacture of a medicament for the treatment and/or prophylaxis of at least one vasopressin-dependent disease.

12. Use of the compound of the formula (I) according to any of Claims **1 to 7** or at least one pharmaceutically acceptable salt or one tautomeric form thereof for the manufacture of a medicament for the treatment and/or prophylaxis of at least one disorder selected from the group consisting of
diabetes, insulin resistance, nocturnal enuresis, incontinence, diseases in which blood coagulation disorders occur,
hypertension, pulmonary hypertension, heart failure, myocardial infarction, coronary spasm, unstable angina, PTCA (percutaneous transluminal coronary angioplasty), ischemias of the heart, disorders of the renal system, edemas, renal vasospasm, necrosis of the renal cortex, hyponatremia, hypokalemia, Schwartz-Bartter syndrome, disorders of the gastrointestinal tract, gastritic vasospasm, hepatocirrhosis, gastric and intestinal ulcer, emesis, emesis occurring during chemotherapy, travel sickness; vasomotor symptoms, thermoregulatory dysfunctions, such as, for example, the "hot flush" symptom;
drug dependencies, medicament dependencies, dependencies mediated by other factors, stress caused by the withdrawal of one or more factors mediating the dependency, stress-induced relapses into the drug dependencies, medicament dependencies and/or dependencies mediated by other factors;
schizophrenia and psychosis;
and/or for delaying micturition.

13. Use of the compound of the formula (I) according to any of Claims **1 to 7** or at least one pharmaceutically acceptable salt or one tautomeric form thereof for the manufacture of a medicament for the treatment of affective disorders, psychoses and/or psychotic disorders, Cushing's syndrome or other stress-dependent diseases and/or sleep disorders.

14. Use of the compound of the formula (I) according to any of Claims **1 to 7** or at least one pharmaceutically acceptable salt or one tautomeric form thereof for the manufacture of a medicament for the treatment of anxiety disorders and/or stress-dependent anxiety disorders.

15. Use of the compound of the formula (I) according to any of Claims **1 to 7** or at least one pharmaceutically acceptable salt or one tautomeric form thereof for the manufacture of a medicament for the treatment of memory impairments and/or Alzheimer's disease.

16. Use of the compound of the formula (I) according to any of Claims **1 to 7** or at least one pharmaceutically acceptable salt or one tautomeric form thereof for the manufacture of a medicament for the treatment of depressive disorders.

17. Use according to Claim **16,** for the manufacture of a medicament for the treatment and/or prophylaxis of childhood onset mood disorders.

18. A compound of the formula (I) according to any of Claims **1 to 7,** or a pharmaceutically acceptable salt or a tautomeric form thereof for use in treatment and/or prophylaxis of a disease or disorder as set forth in any of claims **12 to 17.**

## Patentansprüche

1. Verbindung der allgemeinen Formel (I) 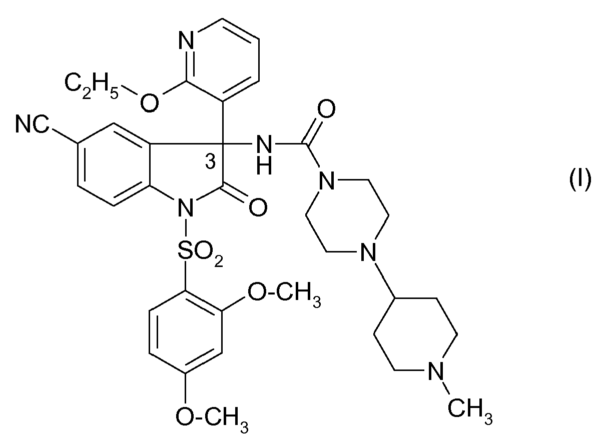 sowie die pharmazeutisch verträglichen Salze und tautomeren Formen davon.

2. Verbindung der Formel (I) nach Anspruch 1, **dadurch gekennzeichnet, dass** sie in optisch aktiver Form vorliegt und dass sie einen Überschuss des Enantiomers aufweist, welches in Form der freien Base die Schwingungsebene von linear polarisiertem Licht nach links dreht,
und die pharmazeutisch verträglichen Salze und tautomeren Formen davon.

3. Verbindung der Formel (I) nach Anspruch 1, **dadurch gekennzeichnet, dass** sie in optisch aktiver Form vorliegt und dass das Enantiomer, worin die absolute Konfiguration des chiralen C-3-Ringkohlenstoffatoms die S-Konfiguration ist, im Überschuss vorliegt.

4. Verbindung der Formel (I) nach Anspruch 2 in optisch aktiver Form, **dadurch gekennzeichnet, dass** das entsprechende linksdrehende (-)-Enantiomer in einer optischen Reinheit (enantiomeric excess, ee) von größer 50 % vorliegt.

5. Verbindung der Formel (I) nach Anspruch 3 in optisch aktiver Form, **dadurch gekennzeichnet, dass** das die bevorzugte absolute Konfiguration am C-3-Ringkohlenstoffatom aufweisende Enantiomer in einer optischen Reinheit (enantiomeric excess, ee) von größer 50 % vorliegt.

6. Verbindung der Formel (I) nach Anspruch 2 in optisch aktiver Form, **dadurch gekennzeichnet, dass** das entsprechene linksdrehende (-)-Enantiomer in einer optischen Reinheit (enantiomeric excess, ee) von größer 90 % vorliegt.

7. Verbindung der Formel (I) nach Anspruch 3 in optisch aktiver Form, **dadurch gekennzeichnet, dass** das die bevorzugte absolute Konfiguration am C-3-Ringkohlenstoffatom aufweisende Enantiomer in einer optischen Reinheit (enantiomeric excess, ee) von größer 90 % vorliegt.

8. Arzneimittel, umfassend eine Verbindung der Formel (I) nach einem der Ansprüche 1 bis 7 oder wenigstens ein pharmazeutisch verträgliches Salz oder eine tautomere Form davon.

9. Verbindung der Formel (I) nach einem der Ansprüche 1 bis 7 oder ein pharmazeutisch verträgliches Salz oder eine tautomere Form davon zur Verwendung als Arzneimittel.

10. Verwendung der Verbindung der Formel (I) nach einem der Ansprüche 1 bis 7 oder wenigstens eines pharmazeutisch verträglichen Salzes oder einer tautomeren Form davon für die Herstellung eines Arzneimittels zur Behandlung und/oder Prophylaxe einer Krankheit oder Störung.

11. Verwendung der Verbindung der Formel (I) nach einem der Ansprüche 1 bis 7 oder wenigstens eines pharmazeutisch verträglichen Salzes oder einer tautomeren Form davon für die Herstellung eines Arzneimittels zur Behandlung und/oder Prophylaxe wenigstens einer Vasopressin-abhängigen Krankheit.

12. Verwendung der Verbindung der Formel (I) nach einem der Ansprüche 1 bis 7 oder wenigstens eines pharmazeutisch verträglichen Salzes oder einer tautomeren Form davon für die Herstellung eines Arzneimittels zur Behandlung und/oder Prophylaxe wenigstens einer Krankheit, die ausgewählt ist unter
Diabetes, Insulinresistenz, Enuresis nocturna, Inkontinenz, Krankheiten, bei denen Blutgerinnungsstörungen auftreten,
Hypertonie, pulmonaler Hypertonie, Herzinsuffizienz, Myocardinfarkt, koronarem Spasmus, instabiler Angina, PTCA (percutaneous transluminal coronary angioplasty), Ischämien des Herzens, Störungen des renalen Systems, Ödemen, renalem Vasospasmus, Nekrose des renalen Cortex, Hyponatriämie, Hypokaliämie, Schwartz-Bartter-Syndrom, Störungen des Gastrointestinaltraktes, gastritischem Vasospasmus, Hepatozirrhose, Magen- und Darmulkus, Emesis, bei der Chemotherapie auftretender Emesis, Reisekrankheit, vasomotorischen Symptomen, thermoregulatorischen Fehlfunktionen, wie beispielsweise das "hot flush"-Symptom, Drogenabhängigkeit, Arzneimittelabhängigkeit, durch andere Faktoren vermittelte Abhängigkeiten, Stress bedingt durch den Entzug von einem oder mehreren die Abhängigkeit vermittelnden Faktoren, Stress-induzierten Rückfällen in die Drogen-, Arzneimittel- und/oder durch sonstige Faktoren vermittelte Abhängigkeiten, Schizophrenie und Psychose;
und/oder zur Verzögerung der Miktion.

13. Verwendung der Verbindung der Formel (I) nach einem der Ansprüche 1 bis 7 oder wenigstens eines pharmazeutisch verträglichen Salzes oder einer tautomeren Form davon zur Herstellung eines Arzneimittels zur Behandlung von affektiven Störungen, Psychosen und/oder psychotischen Störungen, des Cushing-Syndroms oder anderen Stress-abhängigen Erkrankungen und/oder Schlafstörungen.

14. Verwendung der Verbindung der Formel (I) nach einem der Ansprüche 1 bis 7 oder wenigstens eines pharmazeutisch verträglichen Salzes oder einer tautomeren Form davon für die Herstellung eines Arzneimittels für die Behandlung von Angststörungen und/oder stressabhängigen Angststörungen.

15. Verwendung der Verbindung der Formel (I) nach einem der Ansprüche 1 bis 7 oder wenigstens eines pharmazeutisch verträglichen Salzes oder einer tautomeren Form davon für die Herstellung eines Arzneimittels zur Behandlung von Gedächtnisleistungsstörungen und/oder Morbus Alzheimer.

16. Verwendung der Verbindung der Formel (I) nach einem der Ansprüche 1 bis 7 oder wenigstens eines pharmazeutisch verträglichen Salzes oder einer tautomeren Form davon für die Herstellung eines Arzneimittels zur Behandlung depressiver Erkrankungen.

17. Verwendung nach Anspruch 16 für die Herstellung eines Arzneimittels für die Behandlung und/oder Prophylaxe von childhood onset mood disorders.

18. Verbindung der Formel (I) nach einem der Ansprüche 1 bis 7 oder ein pharmazeutisch verträgliches Salz oder eine tautomere Form davon zur Verwendung in der Behandlung und/oder Prophylaxe einer Krankheit oder Störung wie in einem der Ansprüche 12 bis 17 ausgeführt.

## Revendications

1. Composé représenté par la formule générale (I) 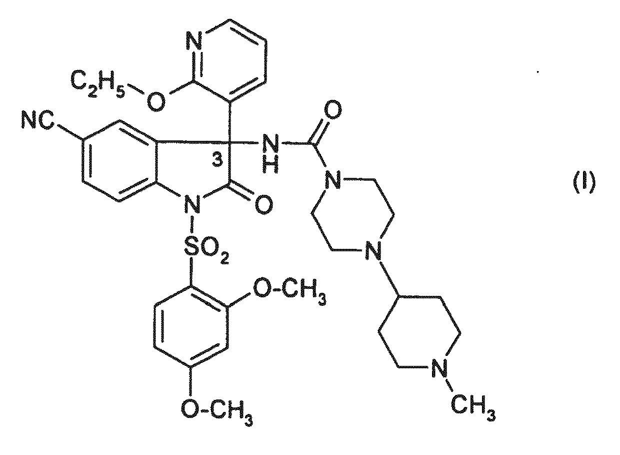 et sels pharmaceutiquement acceptables et formes tautomères de celui-ci.

2. Composé représenté par la formule (I) selon la revendication 1, **caractérisé en ce qu'**il présente une forme optiquement active et **en ce qu'**il comprend un excès de l'énantiomère, qui sous la forme de la base libre fait tourner le plan de polarisation d'une lumière polarisée linéairement vers la gauche,
et sels pharmaceutiquement acceptables et formes tautomères de celui-ci.

3. Composé représenté par la formule (I) selon la revendication 1, **caractérisé en ce qu'**il est présent sous forme optiquement active et **en ce qu'**il comprend un excès de l'énantiomère, dans lequel la configuration absolue de l'atome de carbone de cycle C-3 chiral est la configuration S.

4. Composé représenté par la formule (I) selon la revendication 2 sous forme optiquement active, **caractérisé en ce que** l'énantiomère lévogyre (-) correspondant est présent en une pureté optique (un excès énantiomérique, ee) supérieure à 50 %.

5. Composé représenté par la formule (I) selon la revendication 3 sous forme optiquement active, **caractérisé en ce que** l'énantiomère ayant la configuration absolue préférée au niveau de l'atome de carbone de cycle C-3 est présent en une pureté optique (un excès énantiomérique, ee) supérieure à 50 %.

6. Composé représenté par la formule (I) selon la revendication 2 sous forme optiquement active, **caractérisé en ce que** l'énantiomère lévogyre (-) correspondant est présent en une pureté optique (un excès énantiomérique, ee) supérieure à 90 %.

7. Composé représenté par la formule (I) selon la revendication 3 sous forme optiquement active, **caractérisé en ce que** l'énantiomère ayant la configuration absolue préférée au niveau de l'atome de carbone de cycle C-3 est présent en une pureté optique (un excès énantiomérique, ee) supérieure à 90 %.

8. Médicament, comprenant un composé représenté par la formule (I) selon l'une quelconque des revendications 1 à 7, ou au moins un sel pharmaceutiquement acceptable ou une forme tautomère de celui-ci.

9. Composé représenté par la formule (I) selon l'une quelconque des revendications 1 à 7, ou sel pharmaceutiquement acceptable ou forme tautomère de celui-ci, destiné à être utilisé comme médicament.

10. Utilisation du composé représenté par la formule (I) selon l'une quelconque des revendications 1 à 7 ou d'au moins un sel pharmaceutiquement acceptable ou une forme tautomère de celui-ci pour la fabrication d'un médicament pour le traitement et/ou la prophylaxie d'une maladie ou d'un trouble.

11. Utilisation du composé représenté par la formule (I) selon l'une quelconque des revendications 1 à 7 ou d'au moins un sel pharmaceutiquement acceptable ou une forme tautomère de celui-ci pour la fabrication d'un médicament pour le traitement et/ou la prophylaxie d'au moins une maladie liée à la vasopressine.

12. Utilisation du composé représenté par la formule (I) selon l'une quelconque des revendications 1 à 7 ou d'au moins un sel pharmaceutiquement acceptable ou une forme tautomère de celui-ci pour la fabrication d'un médicament pour le traitement et/ou la prophylaxie d'au moins un trouble choisi dans le groupe constitué par le diabète, l'insulinorésistance, l'énurésie nocturne, l'incontinence, les maladies dans lesquelles surviennent des troubles de la coagulation du sang, l'hypertension, l'hypertension pulmonaire, l'insuffisance cardiaque, l'infarctus du myocarde, un spasme coronarien, l'angine instable, l'ACTP (angioplastie coronarienne transluminale percutanée), les ischémies du coeur, les troubles du système rénal, les oedèmes, le vasospasme rénal, la nécrose du cortex rénal, l'hyponatrémie, l'hypokaliémie, le syndrome de Schwartz-Bartter, les troubles du tractus gastrointestinal, le vasospasme gastrique, la cirrhose du foie, l'ulcère de l'estomac et de l'intestin, les vomissements, les vomissements survenant pendant une chimiothérapie, le mal des transports ;
les symptômes vasomoteurs, les dysfonctionnements de la régulation thermique, tels que, par exemple, le symptôme des « bouffées de chaleur » ;
les toxicomanies, les pharmacodépendances, les dépendances à d'autres facteurs, le stress provoqué par le sevrage d'un ou plusieurs facteurs responsables de la dépendance, les rechutes induites par le stress dans les toxicomanies, les pharmacodépendances et/ou les dépendances à d'autres facteurs ;
la schizophrénie et la psychose ;
et/ou pour retarder la miction.

13. Utilisation du composé représenté par la formule (I) selon l'une quelconque des revendications 1 à 7 ou d'au moins un sel pharmaceutiquement acceptable ou une forme tautomère de celui-ci pour la fabrication d'un médicament pour le traitement de troubles affectifs, de psychoses et/ou de troubles psychotiques, du syndrome de Cushing ou d'autres maladies liées au stress et/ou de troubles du sommeil.

14. Utilisation du composé représenté par la formule (I) selon l'une quelconque des revendications 1 à 7 ou d'au moins un sel pharmaceutiquement acceptable ou une forme tautomère de celui-ci pour la fabrication d'un médicament pour le traitement de troubles anxieux et/ou de troubles anxieux liés au stress.

15. Utilisation du composé représenté par la formule (I) selon l'une quelconque des revendications 1 à 7 ou d'au moins un sel pharmaceutiquement acceptable ou une forme tautomère de celui-ci pour la fabrication d'un médicament pour le traitement de troubles de la mémoire et/ou de la maladie d'Alzheimer.

16. Utilisation du composé représenté par la formule (I) selon l'une quelconque des revendications 1 à 7 ou d'au moins un sel pharmaceutiquement acceptable ou une forme tautomère de celui-ci pour la fabrication d'un médicament pour le traitement de troubles dépressifs.

17. Utilisation selon la revendication 16, pour la fabrication d'un médicament pour le traitement et/ou la prophylaxie de troubles de l'humeur qui se déclarent pendant l'enfance.

18. Composé représenté par la formule (I) selon l'une quelconque des revendications 1 à 7, ou sel pharmaceutiquement acceptable ou forme tautomère de celui-ci, destiné à être utilisé dans traitement et/ou la prophylaxie d'une maladie ou d'un trouble tel qu'indiqué dans l'une quelconque des revendications 12 à 17.
